# EUROPEAN PATENT SPECIFICATION

(11) **EP 1 479 412 B1**
(45) Date of publication and mention of the grant of the patent: **22.10.2008**
(21) Application number: 04252734.1
(22) Date of filing: 12.05.2004
(51) Int. Cl.: A61N 7/02

(54) **Geometrically shaped coupling hydrogel standoffs for high intensity focused ultrasound**
Geometrisch geformte Kopplungskörper aus Hydrogel für die Behandlung mit fokussiertem Ultraschall von hoher Intensität
Corps de couplage d'un hydrogel à forme géométrique pour le traitement par ultrasons focalisés à haute intensité

(30) Priority: 19.05.2003 US 471669 P
(43) Date of publication of application: 24.11.2004
(73) Proprietor: UST Inc., Seattle, Washington 98122-5720 (US)
(72) Inventor: Smith, Larry L., Lummi Island Washington 98262 (US)
(74) Representative: Naylor, Matthew John

(56) References cited:
- EP-A- 0 256 202
- EP-A- 0 344 773
- US-A- 6 039 694
- US-B1- 6 232 406

## Description

### Field of the Invention

The present invention is directed to ultrasound coupling devices and, in particular, to geometrically shaped coupling standoffs consisting of hydrogels for use with high intensity ultrasound.

### Background of the Invention

High Intensity Focused Ultrasound (HIFU) has been reported by many as a means of destroying tissue by thermal means, whereby, the tissue is heated to a temperature that denatures the cell proteins and by mechanical means through disruption of cellular and nuclear membranes caused by localized cavitation. Others have reported the potential for HIFU to rapidly introduce hemostasis (the coagulation of blood and termination of bleeding) during surgery.

The energy requirements for HIFU to cause the therapeutic effects of hemostasis and ablation are on the order of 1,000 to 10,000 Watts/cm². Furthermore, the ultrasound energy most useful for establishing hemostasis and ablation with HIFU is in the frequency range of 2-9MHz, which attenuates quickly in most solid materials including metals and plastics.

it is advantageous, in designing surgical tools based on HIFU, to have the zone of peak ultrasound energy to occur at or near the surface of the surgical tool so that the use is similar to other devices used for coagulation and ablation during surgery. Devices such as the electrocautery knives and argon beam coagulators employ thermal techniques to produce hemostasis and cause ablation at the surface of the surgical tool where it contacts the patient.

One technology for producing high intensity zones useful for hemostasis and ablation is to focus ultrasound energy by means of a lens or curved piezoelectric element. This technique of focusing HIFU requires a coupling medium, typically solid or liquid, between the piezoelectric transducer and the target tissue with sufficient length (typically 1 to 6 cm) to support the transfer of the ultrasound to develop the necessary spatial peak intensity.

A coupling member is an important component of a HIFU surgical device for reasons that include:
1. It is the medium within which acoustic energy is transferred to a point of focus at or in close proximity to the end of the geometric standoff into a small focal zone, typically in the range of 1mm diameter by 6-10mm long, and at high intensity, typically over 1,000 watts/cm².
2. It can be designed so that the focal zone is positioned either at the surface of the distal tip of the coupling member (which contacts the tissue or blood vessel) or beyond the tip at a deeper location in the tissue.
3. It can be sterilized and provided as a disposable that can be replaced during and between surgeries.
4. It must be in vivo biocompatible, as required by its contact with blood and tissue during surgery.

Preferably, a coupling member possesses characteristics that include:
1. Low cost to manufacture into various geometric shapes including but not limited to cones and cylinders.
2. Have low acoustic attenuation in the frequency range of 2-9 MHz enabling efficient coupling of the high intensity focused ultrasound generated by the transducer into the target tissue.
3. Be uniform in acoustic properties so that the acoustic wave generated by the transducer is not distorted in an unpredictable manner by the coupling member.
4. Have an acoustic impedance that is similar to that of tissue and/or blood, thereby allowing the maximum transfer of acoustic energy from the coupling member into the body
5. Be produced from materials that are compatible with tissue and blood for both short and long terms (*in vivo* biocompatible).
6. Be robust in nature, so as to support HIFU with no degradation.
7. Be easily and quickly replaceable during the surgical procedure.

Several materials and techniques have been reported for producing HIFU coupling members. For example:
1. Water
   Water meets all the desired acoustic properties required by a coupling member including the requirement of low attenuation and in vivo biocompatibility. Water is however, difficult to contain in a manner that permits use as a coupling member for a HIFU surgical tool; whereby, the containment method does not in itself alter or negate the desirable characteristics of the water or rupture and cause the device to fail during use with subsequent difficulty in replacing the water coupling member.
2. Metals
   Solid metal, including aluminum or titanium, HIFU coupling cones are robust and have been reported to address the containment problems of water in the construction of HIFU coupling members. Their disadvantages are high manufacturing cost, and high acoustic attenuation and impedance, which results in low energy transfer and the generation of unacceptable amounts of heat in the device.
3. Hydrogels
   Hydrogels offer an attractive combination of the desirable acoustic properties approaching water, as they may be comprised of greater than 60% water, and the advantage of a solid material that does not have the containment problems of water. They are typically moldable, inexpensive to produce and can be quickly changed during a surgical procedure.

Hydrogels have been used as coupling members and specifically as HIFU coupling members. However, hydrogels previously investigated as coupling members were not suitable for use during surgery due to issues of *in vivo* biocompatibilty and/or lack of mechanical strength and resistance to HIFU degradation.

For example, polyacrylamide (PA) has been used as an acoustic coupling member for HIFU. However, polyacrylamide is not an acceptable polymer due to the potential presence of neurotoxic acrylamide monomer in the hydrogel. Acoustic coupling hydrogel standoffs produced from poly (2-hydroxyethylmetacrylate) or pHEMA have been found unsuitable due to their brittle nature and high attenuation, which is also true of hydrogels produced from natural polysaccharides and their derivatives.

US 6232406 discloses hydrogel products formed from polyacrylonitrile copolymers. US 6039694 discloses a coupling sheath comprising polyacrylonitrile block copolymer. EP A 0344773 discloses ultrasonic probes comprising polyurethane gel. EP A 0256202 discloses a solid hydrogel coupling body.

### Summary of the Invention

The present invention provides an acoustic coupling standoff according to claim 1.

The device of this invention relates to an *in vivo* biocompatible hydrogel acoustic coupling standoff for transfer of high intensity ultrasound to achieve hemostasis and ablation during surgery. More specifically, this invention relates to the discovery that a group of hydrogels, based on hydrophilic block co-polymers, one of which is polyacrylonitrile, can form rigid, low acoustic attenuation coupling members and are *in vivo* biocompatible. These inventive devices consist of hydrogel formulations having mechanical and acoustic properties such that ultrasound coupling standoff members of various dimensions and structural configurations such as cones can function as efficient ultrasound transmission media and devices within which the high intensity ultrasound beam can be coupled between the acoustic energy source to a focal point at or in proximity to the standoff terminus. Hydrogel formulations, design and fabrication methods are described for production of ultrasound and energy transmission elements as the device of this invention.

### Brief Description of the Drawings

Figure 1 illustrates a preferred embodiment comprising a geometrically shaped coupling hydrogel standoff in the shape of a cone.

Figure 2 illustrates the geometrically shaped acoustic coupling hydrogel standoff of Figure 1 whereby the acoustic coupling member is contained within a external retention capsule which is attached to a transducer housing.

### Detailed Description of the Preferred Embodiment

Discussed below is the formulation, design and fabrication of hydrogels that possess the acoustic, mechanical and structural properties required to function as ultrasound coupling and transmission media as is used in high intensity focused ultrasound (HIFU) applications such as hemostasis and ablation during surgery. Percentages, where given, are weight percentages.

The present invention is preferably directed to geometrically shaped HIFU coupling members that are suitable for acoustic hemostasis and ablation within the human body. Preferably, the coupling members exhibit properties that include:
- *in vivo* biocompatibility;
- low acoustic attenuation at HIFU frequencies;
- ability to be easily molded into shapes;
- relatively low manufacturing cost;
- acoustic impedance similar to that of tissue and blood;
- relatively robust, not brittle, durable during the surgical and HIFU procedure;
- easy to replace during or between surgical procedures;
- sterilizable.

Selection of hydrogels for coupling elements was based on polymer *in vivo* biocompatibility with subsequent evaluation of conformance to mechanical and acoustic property requirements necessary to form and function. High intensity focused ultrasound (HIFU) utilizes high frequency sound, typically between 2 and 9 MHz. Acoustic energy at such frequencies is poorly transmitted by air and requires an acoustic coupling member, typically a solid or liquid, between the transducer and the tissue. Acoustic coupling media have commonly been fluids, gels, or solids to efficiently transfer the acoustic energy between the HIFU applicator and the target tissue. As examples, Larson et al. in U.S. Patent.No. 6,039,694 teach the use of an in vivo biocompatible hydrogel as an acoustic couplant in membrane form while Montecalvo in U.S. Patent No. 5,522,878 teaches the use of hydrogel in sheet form for coupling ultrasound energy which, however, by virtue of its chemical composition is not in vivo biocompatible. Martin et al. in U.S. Patent No. 6,432,067 teach the use of water or other acoustic transmissive media contained inside a flexible membrane such as polyurethane to couple the ultrasound energy. In U.S. Patent No. 6,217,530 Martin et al. teach the use of solid acoustic coupling standoffs composed of ceramic, glass and metals for transmitting the HIFU energy.

The inventive hydrogel acoustic coupling element operates as a geometric standoff between the transducer and the object of therapy and overcomes the limitations and deficiencies of the inventive devices of Martin et al. As used in HIFU applications, the high frequency acoustic energy is concentrated into a small volume (typically in the shape of a grain of rice 7-10 mm in length) and at high intensity (typically over 1,000 watts/cm²). The hydrogels thus used for such ultrasound energy transmission must provide low levels of attenuation to limit heating within the coupling element, and efficiently transfer the energy to the treatment site. The hydrogel thus used must also be thermally robust at the HIFU acoustic intensities, be *in vivo* biocompatible, relatively inexpensive, sterilizable and moldable into various geometries, such as cones.

The polymers are polyacrylonitrile block co-polymers that are sequenced with hydrophobic nitrile blocks in series with attached hydrophilic units that function as reaction sites. The length of the crystalline polyacrylonitrile groups and variability of species and concentration of the hydrophilic side groups, for example, such compounds as acrylic acid, acryl amids, and acrylamidine, together with modifications to the manufacturing process, produce polymers that demonstrate a range of physical and mechanical properties suited for use as focus elements for HIFU applications. The polyacrylonitrile co-polymer used in the device of this invention produces cohesive strengths that approximate the energy levels of covalent, cross-linked hydrogels. These attributes produce hydrogels with acceptable toughness, tensile, elongation, and tear strength properties.

This family of block co-polymers can be formulated so as to provide water or saline contents ranging from about 70% to about 95%. Presence of water or saline in the structure at these levels of concentration, provide an acceptable low levels of attenuation as is required for efficient energy transfer. The polyacrylonitrile copolymers melt at temperatures in excess of 150° C and thus provide thermal stability at operating temperatures that can reach 100° C.

The polymers of the preferred embodiment are hydrophilic acrylic acid derived block co-polymers such as those described in U.S. Patents No. 5,252,692 to Lovy et al. and 6,232,406 to Stoy. The most preferred polymer is based on a family of hydrophilic acrylate derivatives which as a molecule is structured such that a backbone of hydrophobic polyacrylonitrile groups are sequenced with a series of attached hydrophilic units that function as reaction sites. The size of the nitrile groups and variability of the hydrophilic side groups provide opportunities to tailor the polymer to produce mechanical properties that are optimum for various applications.

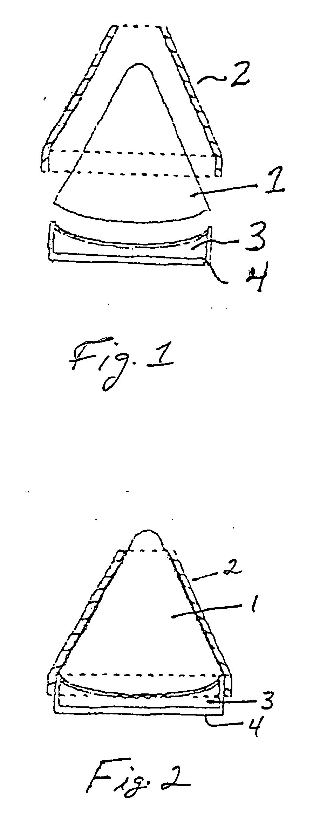

As shown in Figures 1 and 2, the device of an embodiment of this invention is an acoustic coupling hydrogel standoff 1 which is a solid free standing hydrogel coupling member requiring no restraint or alternatively held within a retainer 2 for secure attachment and intimate contact interface to the face of a transducer 3 and its housing 4.

Hydrogel focus cones for HIFU applications, which are the preferred embodiment, are produced from hydrophilic acrylic acid block co-polymers in the form of "pre-polymer" solutions being so composed as to contain polymer melted in solvents such dimethylsulfoxide, dimethylformamide and water solution containing 55 % sodium thiocyanate. Solvent concentrations are typically present in amounts of up to 55% with 2% to 35% block co-polymer, preferably about 5% to about 12% and most preferably about 8% to about 12%, comprising the above mentioned acrylate derivatives, nitrile groups and hydrophilic side groups. Such polymer solutions are known and commercially available.

Manufacture of the inventive hydrogel focus cones is most efficiently accomplished by casting the pre-polymer into molds prepared from porous ceramics, chemically compatible polymers and stainless steel. Alternatively, manufacturing can take the form of dipping, rotational casting or extrusion. Hydrogels of this family are formed by replacement of the polymer solvent during coagulation in water. Water solvates the soft blocks of the polymer and precipitates the hard hydrophobic blocks thus forming a new phase, which results in solidification of the polymer.

The acoustic coupling devices of this invention are preferably cast into porous molds as an 8-15 % solution of the copolymer in aqueous 55 % sodium thiocyanate. Prevention of gas bubbles in the finished coupling element is important to transmission without attenuation or scattering of acoustic energy through the hydrogel focus element. To accomplish removal of entrapped gasses in the pre-polymer solution, the solution can be sealed under a nitrogen gas blanket, heated to 55° C and held at constant temperature for 8 hours prior to casting. This solution can then be drawn into a syringe or production device suitable for bottom up filling of the mold to exclude formation of macro bubbles. The filled molds are allowed to stand for sufficient time to allow any entrapped air to rise to the surface of the polymer or casting vent.

Since the polymer is dissolved in a solvent, in this case preferably sodium thiocyanate, or DMSO or dimethylformamide, such solvents must be removed or extracted to permit formation of the desired hydrogel structure. This process of extraction, described as coagulation, is accomplished by submersion of a water porous mold and its contents into a water, or saline (0.45% to 0.9% NaCl) bath at 45°C followed by continuous rinsing with water until the solvent is removed and coagulation is complete. Completion time of solvent extraction and complete coagulation of the polymer solution is influenced by the thickness of the casting, the temperature of the rinse water and the concentration of the polymer solvent in the bath.

The coupling element castings are coagulated by rinsing in water or, preferably, saline (0.45% to 0.9% NaCl), with repeated changes of rinse water, until the rinse water contains only acceptably low trace amounts of the solvent such as NaSCN, which can be determined by conductivity testing. The final dimensions of the cast hydrogel coupling elements can be adjusted by changes to the salinity of the final rinse and storage solution. As salinity is varied, mechanical properties and dimensions change. As the salinity of the storage solution is increased, the hydrogel shrinks dimensionally, and the tensile strength increases together with a decrease in elasticity. As the salinity is decreased the converse is true. Once coagulated and stabilized at desired salinity, the acoustic coupling standoff members can be removed from the molds and further processed for packaging. Sterilization is best accomplished by E-beam (electron beam), or gamma radiation.

Complex shapes with exacting dimensional specifications, such as the device of this invention, are best formed by compression molding. In such case, the polymer solution is enclosed in molds that are porous and can to advantage use the positive osmotic pressure created by a greater permeation of water from the rinse bath than outflow of the solvent in the polymer solution. This differential forms positive osmotic pressure within the mold, such that the coagulating gel swells and conforms to the shape of the mold thus providing for exacting shapes and dimensions.

By design, the cast hydrogel coupling elements, such as cone shapes, are configured so that the base of the acoustic coupling element physically and intimately conforms to the contours of the transducer face. In practice, the HIFU coupling members of this invention are secured to the transducer face so as to maintain a conformal and air free interface between the two. Such conformal interface produces an acoustic coupling between the ultrasound transducer and the hydrogel HIFU coupling member, thus providing for the transmission of the ultrasound energy at or proximate to the site of device contact with tissue, blood or blood vessels.

The mechanical and structural requirements imposed by rigid self-supporting hydrogel focus members limit the selection of suitable polymers for HIFU applications. Hydrogel acoustic coupling standoffs, when designed so as to incorporate use of acoustically transparent shells or containment devices, allow other polymers to become candidates. Such acoustically transparent devices can function as molds in the casting process and/or as a retainer device when in use during therapy.

Polymers suitable for producing such modified hydrogel coupling standoffs by use of a retainer shell include hydrogels that form high viscosity gels and semi-solids, generally produced by covalent cross-linking or thru application of e-beam or gamma radiation, such as is in the case of high energy cross-linked PEO.

While this invention has been described with reference to medical or therapeutic ultrasound applications with human tissue as a target, and in particular to surgical methods, it is not to be limited thereto.

## Claims

1. An in vivo biocompatible hydrogel acoustic coupling standoff (1) for transferring high intensity ultrasound, **characterized in that** said standoff is a free-standing cone, truncated cone, angular truncated cone, oval cone or oviform cone, with said hydrogel comprising polyacrylonitrile block co-polymer.

2. The standoff (1) of claim 1 wherein said hydrogel comprises crystalline polyacrylonitrile and acrylic acid compounds.

3. The standoff (1) of any one of the previous claims further comprising about 70 wt.% to about 95 wt.% water or saline.

4. The standoff (1) of any one of the previous claims wherein said standoff is solid.

5. The standoff (1) of any one of the previous claims wherein said standoff includes a base surface.

6. The standoff (1) of claim 5 wherein the standoff is secured to a face of an ultrasound transducer (3), the base surface of said standoff being in conformance to and in contact with the transducer face.

7. The standoff (1) of claim 6 further including an acoustically transparent retainer device (2) arranged thereabout.

8. The standoff (1) of claim 6 or 7 being removable and replaceable.

9. The standoff (1) of any one of the previous claims being sterilizable.

10. Use of an in vivo biocompatible hydrogel for manufacturing an acoustic coupling standoff (1) according to any one of claims 1 to 9.

## Patentansprüche

1. Biokompatibler, akustischer In-vivo-Kopplungskörper aus Hydrogel (1) zum Weiterleiten von Ultraschall von hoher Intensität, **dadurch gekennzeichnet, dass** der Körper ein freistehender Kegel, ein Kegelstumpf, ein winkeliger Kegelstumpf, ein ovaler Kegel oder eiförmiger Kegel ist, wobei das Hydrogel ein Polyacrylnitril-BlockCopolymer umfasst.

2. Körper (1) nach Anspruch 1, worin das Hydrogel kristallines Polyacrylnitril und Acrylsäurekomponenten umfasst.

3. Körper (1) nach einem der vorangegangenen Ansprüche, ferner umfassend ungefähr 70 Gew.-% bis ungefähr 95 Gew.-% Wasser oder Saline.

4. Körper (1) nach einem der vorangegangenen Ansprüche, worin der Körper fest ist.

5. Körper (1) nach einem der vorangegangenen Ansprüche, worin der Körper eine Basisoberfläche umfasst.

6. Körper (1) nach Anspruch 5, worin der Körper an einer Fläche eines Utraschallwandlers (3) befestigt ist, wobei die Basisoberfläche des Körpers in Übereinstimmung mit und in Kontakt mit der Wandlerfläche ist.

7. Körper (1) nach Anspruch 6, ferner umfassend eine akustisch transparente Haltevorrichtung (2), die um diesen angeordnet ist.

8. Körper (1) nach Anspruch 6 oder 7, welcher entfernbar und ersetzbar ist.

9. Körper (1) nach einem der vorangegangenen Ansprüche, welcher sterilisierbar ist.

10. Verwendung von biokompatiblem In-vivo-Hydrogel zur Herstellung eines akustischen Kopplungskörpers (1) nach einem der Ansprüche 1 bis 9,

## Revendications

1. Corps de couplage acoustique en hydrogel biocompatible in vivo (1) pour transférer un ultrason de forte intensité, **caractérisé en ce que** ledit corps de couplage est un cone autonome, un cone tronqué, un cone angulaire tronqué, un cone ovale ou un cone oviforme, ledit hydrogel comprenant un copolymère séquencé de polyacrylonitrile.

2. Corps (1) de la revendication 1 où ledit hydrogel comprend du polyacrylonitrile cristallin et des composés d'acide acrylique.

3. Corps (1) de l'une quelconque des revendications précédentes comprenant de plus environ 70% en poids à environ 95% en poids d'eau ou d'une solution saline.

4. Corps (1) de l'une quelconque des revendications précédentes où ledit corps de couplage est solide.

5. Corps (1) de l'une quelconque des revendications précédentes où ledit corps de couplage comprend une surface de base.

6. Corps (1) de la revendication 5 où le corps de couplage est fixé à une face d'un transducteur d'ultrasons (3), la surface de la base dudit corps de couplage étant en conformité avec et en contact avec la face du transducteur.

7. Corps (1) de la revendication 6, comprenant de plus un dispositif de retenue acoustiquement transparent (2) agencé autour de lui.

8. Corps (1) de la revendication 6 ou 7 étant amovible et remplaçable.

9. Corps (1) de l'une quelconque des revendications précédentes étant stérilisable.

10. Utilisation d'un hydrogel biocompatible in vivo pour la fabrication d'un corps de couplage acoustique (1) selon l'une quelconque des revendications 1 à 9.
